# EUROPEAN PATENT APPLICATION

(11) **EP 3 061 822 A1**
(43) Date of publication of application: **31.08.2016**
(21) Application number: 15156547.0
(22) Date of filing: 25.02.2015
(51) Int. Cl.: C12N 15/10, C12Q 1/68

(54) **Method for concentrating, isolating and/or purifying nucleic acids from highly diluted aqueous samples**

(71) Applicant: QIAGEN GmbH, 40724 Hilden (DE)
(72) Inventor: Hucklenbroich, Jörg, 42929 Wermelskirchen (DE); Singer, Thorsten, 42699 Solingen (DE)
(74) Representative: f & e patent

(57) **Abstract**

The present invention relates to a method for concentrating, isolating and/or purifying nucleic acids from highly diluted aqueous samples, comprising said nucleic acids in a concentration of less than 500 ng/mL, by means of chaotrope-free binding to a solid phase in the presence of a binding mediating agent, the use of specific cationic surfactants as binding mediating agents in such a method, as well as to a kit for carrying out said method.

## Description

The present invention relates to a method for concentrating, isolating and/or purifying nucleic acids from highly diluted aqueous samples, comprising said nucleic acids in a concentration of less than 500 ng/mL, by means of chaotrope-free binding to a solid phase in the presence of a binding mediating agent, the use of specific cationic surfactants as binding mediating agents in such a method, as well as to a kit for carrying out said method.

Various methods are known for the isolation of nucleic acids like DNA and RNA from aqueous samples comprising said nucleic acids, e. g. biological samples lysed in an aqueous lysis buffer. However, many of them are not suitable for samples comprising a very low concentration of the nucleic acids of interest, i. e. less than, for examples, 500 ng/mL. Such samples may comprise for example extra-cellular nucleic acids, also called free-circulating nucleic acids, in cell-free body fluids such as urine. On the other hand, in particular these free-circulating nucleic acids are often of great interest, for example as biomarkers for cancer or disease states.

At present, commonly used methods suitable for concentrating, isolating and/or purifying nucleic acids from low concentrated aqueous samples are usually based on their adsorption at surfaces made of glass or silica in presence of a rather high concentration of chaotropic salts, followed by the elution from said surfaces, as first described by Vogelstein and Gillespie (Vogelstein, B. Gillespie, Proc. Nat. Acad. Sci. USA 1979, 76, 615 - 619). The chaotropic salts used in these methods are toxic and/or corrosive, but nevertheless usually required in a rather high concentration for binding. This makes these methods unfavorable in particular for concentrating highly diluted solutions of nucleic acids, wherein rather large sample volumes have to be processed. Moreover, some of these methods perform rather poor in the isolation of nucleic acids fragments having a length of less than 100 base pairs (bp) or nucleotides (nt) for DNA and RNA, respectively. Since on the other hand free-circulating nucleic acids usually are present as short fragments (<1.000 bp/nt) including free-circulating miNRA as small as 20 nt, there is a need for a method for concentrating, isolating and/or purifying nucleic acids from highly diluted aqueous samples, which can be automated and is as fast as the chaotrope-based methods known in the state of the art, but on the other hand avoids the use of these toxic substances.

This object is met by the method of the present invention. The present invention provides a method for concentrating, isolating and/or purifying nucleic acids from an aqueous sample comprising said nucleic acids in a concentration of less than 500 ng/mL, preferably less than 250 ng/mL, more preferably less than 150 ng/mL and most preferably equal to or less than 100 ng/mL, said method comprising a step of separating the nucleic acids from the aqueous sample by contacting the sample with a binding mediating agent and a solid phase which selectively binds the nucleic acids in the presence of the binding mediating agent, wherein neither the binding mediating agent nor any other compound added to the sample comprises a chaotropic substance, selected from the group comprising sodium perchloride, sodium iodide, potassium iodide, guanidinium thionate, guanidinium isothiocyanate and guanidine hydrochloride.

The method of the present invention allows for the concentration, isolation and/or purification of nucleic acids from large sample volumes, e. g. more than 20 mL, of highly diluted aqueous samples comprising nucleic acids, even for samples comprising only small fragments (below 100 bp/nt) of nucleic acids. Thus, the method of the present invention combines the known advantages of solid phase-based separation technology with the further advantage that no toxic and/or corrosive chaotropic substances have to be employed during the whole extracting procedure. This is possible, since it has surprisingly been found that a non-chaotropic binding mediating agent is able to selectively mediate the binding between the solid phase and the nucleic acids even in highly diluted solution.

If the aqueous sample comprises any solid material, such as for example cells, cell debris or precipitates from previous purification steps, these solids may optionally be separated from the aqueous sample in a first step by any of the conventional methods for removing a solid from a liquid known in the state of the art, preferably for example by centrifugation or filtration.

In terms of the present invention a binding mediating agent is a non-chaotropic substance which mediates selective binding between the solid phase and the nucleic acids.

By "mediating selective binding" it is meant, that in the absence of the binding mediating agent the nucleic acids do not bind to the solid phase or at least only in a very limited extent (i. e. less than 2 %, preferably less than 1 %, more preferably less than 0,5 % of all nucleic acids present in the sample are bound to the solid phase). On the other hand, in presence of said binding mediating agent the nucleic acids present in the sample bind/attach to the solid phase, for example via ionic or electrostatic interactions in increased amounts, whereas other compounds present in the sample, such as for example salts etc. are essentially not bound to the solid phase in a relevant amount (i. e. less than 2 %, preferably less than 1 %, more preferably less than 0,5 % of any such substances bind to the solid phase), except for the binding mediating agent.

Chaotropic substances effect denaturation of proteins, increase the solubility of non-polar substances in water and destroy hydrophobic interactions. In addition, they also mediate the binding of the nucleic acids to selected solid phases. The Hofmeister series (also called lyotropic series) provides a ranking for the relative affect of ions on the physical behaviour of water and aqueous processes, such as colloidal assembly or protein folding:
CO₃²⁻ > SO₄²⁻ >S₂O₃²⁻ > H₂PO₄⁻ > F⁻ > Cl⁻ > Br⁻> NO₃⁻ > I⁻ > ClO₄⁻ > SCN⁻ [for anions] and
NH₄⁺ > K⁺ > Na⁺ > Li⁺ > Mg²⁺ > Ca²⁺ > guanidinium [for cations].

"Early" members of the series, i.e. members on the left side, increase the surface tension of the solvent and decrease the solubility of non-polar molecules, such as proteins (so-called "salting out"/aggregation), while the "later" members (on the right side) decrease the solvent's surface tension and increase the solubility of non-polar molecules (so-called "salting in"/solubilizing). The specific order of two ions in the series may vary depending from the scientific reference, in particular for ions positioned in the middle of the series, it is however generally accepted that iodide, perchlorate, thiocyanate, and/or guanidinium ions are chaotropic ions, while sulphate and ammonium ions, for example, are non-chaotropic ions. Thus, in terms of the present invention a chaotrope in particular is a water-soluble salt comprising iodide, perchlorate, thiocyanate, and/or guanidinium ions, such as for example sodium perchlorate, sodium iodide, potassium iodide, guanidinium thiocyanate, and guanidine hydrochloride.

In the method of the present invention these chaotropic substances are not required for selectively binding the nucleic acid(s) to the solid phase in order to essentially separate the nucleic acids from the sample. Thus, preferably neither the binding mediating agent nor any other compound or component added to the sample during the procedure comprises a significant amount of a chaotropic substance selected from the group comprising sodium perchlorate, sodium iodide, potassium iodide, guanidinium (iso)thiocyanate, and guanidine hydrochloride. In terms of the present invention, a significant amount of a chaotropic substance preferably is an amount of 1 M or more, more preferably of 0.5 M or more, even more preferably of 0.1 M or more, and most preferably of 0.05 M or more. Preferably, neither the binding mediating agent nor any other compound or component added to the sample during the procedure comprises any chaotropic substance. It is further preferred, that the initial aqueous sample does not comprise more than 1 M, preferably not more than 0.5 M, more preferably not more than 0.1 M, and even more preferably not more than 0.05 M of any of the aforementioned chaotropes. Most preferably the sample does not comprise any chaotropes at all.

To increase the yield of nucleic acids from the sample comprising nucleic acids in any packaged or enveloped form (e.g. comprised in cells, in a virus or any other possible occurring form), it is possible to carry out an optional lysis step, allowing the release of the nucleic acid from the surrounding cover. For lysis preferably a lysis buffer or method is used which doesn't involve the addition of any chaotropic compound as defined above to the sample. Examples for compounds or methods suitable to carry out said lysis step are the addition of a protease to the sample, the application of pressure or expansion, the addition of detergents or similar, without being limited to these.

After the optional step of removing solids from the sample, the sample may be contacted with the solid phase and optionally may be incubated in contact with said solid phase. The solid phase comprising the bound nucleic acid may then be separated essentially from any remaining parts of the sample not bound to the solid phase, such as for example salts, proteins, etc. To complete separation of the nucleic acid bound to the solid phase from any remaining contaminants the solid phase optionally may be washed. After washing, it may optionally be dried, before the nucleic acid preferably may be eluted from the solid phase.

Preferably, the method of the present invention comprises a step of mixing the sample with said binding mediating agent before or while contacting the sample with the solid phase. E. g. the sample may be contacted with the solid phase which is already present in the solution of binding mediating agent, a mixture of the aqueous sample and the binding mediating agent may be applied to the solid phase, the aqueous sample and the binding mediating agent may be simultaneously applied to the solid phase, or the binding mediating agent may be added to a mixture of the aqueous sample and the binding mediating agent.

The binding mediating agent may preferably comprise at least one organic cation, preferably selected from the group comprising cationic polyamines and cationic surfactants. Preferably cationic polyamines obtained by protonation of polyamines of the general formula R¹R²N[(CH₂)₂₋₆NR³]ₙ(CH₂)₂₋₆NR⁴R⁵) may be applied, wherein R¹, R², R³, R⁴ and R⁵ may be the same or different, and independently represent hydrogen or a C₁-C₂₀ alkyl group, more preferably hydrogen or a C₃-C₆ alkyl group, and n represents an integer ranging from 0 to 6. If n > 1, the polyamines comprise more than one subunit of the formula [(CH₂)₂₋₆NR³], and R³ may be the same or different in each of these subunits. Preferably polyamines are applied which are cationic at any pH in the range of from pH 1 to pH 14, such as spermine or spermidine.

Preferred cationic surfactants may comprise the general structure R¹R²R³R⁴N⁺X⁻, wherein R¹, R², R³, and R⁴ independently represent a C₁-C₂₀ alkyl group and X⁻ represents a negatively charged counter ion. The negatively charged counter ion can be organic or inorganic, and preferably may be an inorganic counter ion, most preferably representing Cl- or Br-.

Preferably R¹ may represent a linear C₈-C₁₆-alkyl chain, in particular n-octyl, n-nonyl, n-decyl, n-undecyl, n-dodecyl, n-tridecyl, n-tetradecyl, n-pentadecyl, and n-hexadecyl and R², R³, and R⁴ independently may represent methyl (Me) or ethyl (Et). Preferably the cationic surfactant may be selected from the group comprising octyltrimethylammonium bromide (OTAB), dodecyltrimethylammonium bromide (DTAB), tetradecyltrimethylammonium bromide (TTAB), and cetyltrimethylammonium bromide (CTAB) or a mixture thereof, and most preferably represents CTAB.

According to the present application a definition of e. g. C₁-C₂₀ means any amount of C-atoms between C₁ and C₂₀, i. e. C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀. The binding mediating agent may be provided in form of an aqueous solution. Preferably said aqueous solution which is to be added to the sample comprises from about 0.001 to about 5 wt.-% of one or more binding mediating agent(s) mentioned above, preferably from about 0.005 to about 1 wt.-% and most preferably from about 0.01 % to about 0.2 %. In contrast to chatrope mediated binding known from the state of the art, in the present invention rather small concentrations of binding mediating agent in the sample are sufficient for selectively and (almost) quantitatively binding the nucleic acids to the solid phase. In the sample solution which is obtained after adding the binding mediating agent typically a concentration of less than 1 M, preferably less than 0.1 M, even more preferably less than 0.05 M, and most preferably less than 0.02 M of the binding mediating agent in the sample may be used. For example a concentration about 0.01-0.02 wt.-% CTAB (corresponding to 5.5 mM/L) in the sample solution is sufficient to selectively and essentially quantitatively bind nucleic acid fragments of all different sizes to a nucleic acids binding solid phase. Preferably said aqueous binding mediating agent solution may comprise less than 2 M of any inorganic salt, more preferably less than 0.5 M, even more preferably less than 0.1 M, and most preferably said binding mediating agent solution is free of any inorganic salt. Preferably said binding mediating agent solution may comprise less than 20 % of any alcohol, more preferably less than 5 %, even more preferably less than 1 % and most preferably said binding mediating agent solution does not comprise any alcohol at all.

The solid phase may be selected from the group comprising beads, including magnetic beads, particulates, powders, layers, meshes, tips, dipsticks, tubes, container walls, filters, gels, ion exchange resins, reversed phase resins, matrices, and membranes, preferably from the group comprising polystyrene or silica beads, magnetic beads coated with a polystyrene or silica layer, polystyrene or silica beads further functionalized with negatively charged polymers, polystyrene or silica slides, polystyrene or silica powder, quartz filter paper, quartz wool, polystyrene or silica membranes, and polystyrene or silica matrices, and most preferably may represent a polystyrene or silica membrane or a polystyrene or silica matrix. These most preferred embodiments allow a fast and automatic handling of a plurality of samples in high throughput processing. In principle, any solid phase suitable for binding nucleic acids in the presence of a high concentration of chaotropic substances may be used in the method for the present invention, a plurality of which is well known to a person skilled in the art.

After binding the nucleic acids to the solid phase, at least one, preferably two or more washing steps may be carried out to remove contaminants from the nucleic acids bound to the solid phase. Preferably these washing steps may be carried out by using washing liquid(s)/washing buffer(s) selected from the group comprising water, aqueous solutions comprising 0.01 to 25 % (v/v) of a C₁-C₅-alcohol including methanol, ethanol and isopropanol, a buffering agent, a chelating agent or mixtures thereof, e. g. acidic and basic buffering solutions, such as for example commercially available TRIS, MES, MOPS, or HEPES buffer, or low salt washing buffers, such as for example the commercially available buffer PE (Qiagen, Hilden, Germany), without being limited to these. Optionally said washing liquid(s)/washing buffer(s) may comprise at least in the first washing step the binding mediating agent as described above.

For example, after binding the nucleic acids to the solid phase, the solid phase may first be washed using nuclease-free water or the commercially available buffer PE (Qiagen, Hilden, Germany) followed by a second washing step using an aqueous solution comprising a C₁-C₅ alcohol, including methanol, ethanol and iso-propanol in a concentration of ≥ 80 % (v/v). The step of washing with a concentrated alcohol solution removes the binding mediating agent from the solid phase while simultaneously precipitating the nucleic acids on the solid phase. Accordingly, it is preferred to quickly carry out this particular washing step, i.e. in about 15 sec to 1.5 min, preferably at a high centrifugal force of about 10,000 xg or above.

If CTAB is used as binding mediating agent the nucleic acids may be eluted from the solid phase using aqueous solutions of ethanol, comprising 30 wt.-% or more of ethanol. The unintended elution of nucleic acids bound to the solid phase during ethanol washes for removing CTAB can be prevented by a fixative wash, using an aqueous solution comprising about 15 to 25 % of a C₁-C₅ alcohol, including methanol, ethanol, n-propanol, iso-propanol, n-butanol, and n-pentanol, preferably ethanol and about 3 to 10 mM, preferably about 5 to 7 mM spermine or spermidine. After this fixative wash, the nucleic acid may be eluted e. g. by using pure water, or preferably a low salt solution / buffer having a salt concentration of 500 mM or less, preferably 250 mM or less, more preferred 100 mM or less, even more preferred 50 mM ore less, however, having at least 0.1 mM, preferably 1 mM more preferred 10 mM of a suitable salt, (e.g. NaCl), if spermidine had been used as a fixative. Such a low salt solution preferably has a conductivity of at least about 60 mS/m, if spermine had been used as a fixative. In an acidic environment (pH < 7) binding of the nucleic acids to the solid phase is stronger than in an alkaline environment (pH > 7), and a higher amount of eluting agent (and/or a higher concentration of the eluting substance(s) therein) is necessary.

If a washing step using an aqueous alcoholic solution has been employed, it may be preferred to optionally dry the solid phase, for example by incubating the solid phase at room temperature, dry centrifugation, incubating at elevated temperature and/or reduced pressure or any similar suitable method, before eluting the nucleic acids from the solid phase.

A further advantage of the method of the present invention is the fact that the step of eluting the nucleic acids from the solid phase may be carried using a low salt eluting buffer or even water. Thus, the step of eluting the nucleic acids from the solid phase according to step g) may be carried out using an aqueous solution comprising less than 1 M of salt. In principle, any solution not precipitating nucleic acids may be used. Preferably the solution comprises one or more buffering agents, selected from the group comprising Tris-HCl, MOPS, MES or HEPES and optionally a chelating agent, preferably selected from the group comprising EDTA, EGTA and citrate without being limited to these. A typical elution buffer may for example comprise about 10 mM of a buffering substance and about 1 mM of a chelating agent, adjusted to pH 7.5 - 9. In principle, all low salt eluting buffers known from the state of the art may be used in the present invention, provided that they do not comprise any of the chaotropic substances mentioned above.

Using the method of the present invention RNA, DNA, as well as engineered nucleic acids such as protein-nucleic acids (PNA) and locked nucleic acids (LNA) can be extracted from the respective aqueous samples. Preferably the nucleic acids may be DNA, including eukaryotic, prokaryotic and viral DNA, preferably selected from the group comprising genomic DNA, amplified DNA, expressed DNA, bio-engineered DNA, episomal DNA, plasmid DNA, mitochondrial DNA, cDNA, or a fragment thereof or a mixture thereof. Further microRNA (mi-RNA), RNAi or RNA or RNA fragments of RNA viruses are also preferred targets.

In a particularly preferred embodiment, the binding mediating agent may represent CTAB and the step of eluting the nucleic acids from the solid phase according to step g) may be carried out using an aqueous salt solution (e.g. the low salt solution / buffer mentioned above), having a conductivity of at least 60 mS/m determined according to standard methods or alcoholic solutions comprising at least 30 % (v/v) of an alcohol, preferably ethanol. If the solid phase is a silica-based solid phase, a low salt sodium chloride buffer may be preferred, whereas in the case of polystyrene based solid phases, elution with magnesium chloride solutions may be preferred.

Furthermore, the invention relates to the use of a cationic surfactant of the formula R¹R²R³R⁴N⁺X⁻, wherein R¹ represents a linear C₈-C₁₆-alkyl chain and R², R³, and R⁴ independently represent Me or Et, preferably selected from the group comprising octyltrimethylammonium bromide (OTAB), dodecyltrimethylammonium bromide (DTAB), tetradecyltrimethylammonium bromide (TTAB), and cetyltrimethylammonium bromide (CTAB) or a cationic polyamine, preferably selected from spermine und spermidine, or a mixture thereof in a method for concentrating, isolating, and/or purifying nucleic acids from an aqueous liquid, comprising said nucleic acids in the concentration of less than 500 ng/mL preferably less than 250 ng/mL, more preferably less than 150 ng/mL and most preferably equal to or less 100 ng/mL.

Using the method of the present invention RNA, DNA, as well as engineered nucleic acids such as protein-nucleic acids (PNA) and locked nucleic acids (LNA) can be extracted from the respective aqueous samples. Preferably the nucleic acids may be DNA, including eukaryotic, prokaryotic, yeast and viral DNA, preferably selected from the group comprising genomic DNA, amplified DNA or RNA, expressed DNA, bio-engineered DNA, episomal DNA, plasmid DNA, mitochondrial DNA, cDNA, mi-RNA, RNAi or viral RNA or a fragment thereof or a mixture thereof. Preferably, the nucleic acids are extra-cellular nucleic acids, i. e. circulating nucleic acids, preferably free-circulating nucleic acids, including DNA and RNA, and most preferably free-circulating DNA. In principal, the aqueous sample to be processed according to the method of the present invention may be any sample comprising a low concentration of nucleic acids i. e. less than 500 ng/mL. It is, however, preferred, that the aqueous sample comprises less than 1µg/mL, preferably less than 100 mg/mL, more preferred less than 10 ng/m, even more preferred less than 1 ng/mL and most preferred no protein. More preferably, the aqueous sample is a cell-free body fluid like plasma, serum, liquor urine or tears liquid, most preferably a body fluid of low protein content, and in particular represents urine.

All types of DNA / RNA might be be concentrated, isolated and/or purified according to the present invention, from small oligonucleotides e.g. having 5 or more nucleotides (nt) or base pairs (bp) up to genomic DNA having more than 50.000 bp per molecule. The length of the nucleic acids preferably may be in the range of 10 bp (DNA)/nt (RNA) to 10.000 bp (DNA)/nt (RNA), more preferably of from 20 bp (DNA)/nt (RNA) to 5.000 bp (DNA)/nt (RNA), even more preferred from 30, 40, 50, 100, 200 or 500 or any number inbetween to 1.000, 2.000, 3.000 or 4.000, or any number inbetween the particularly mentioned limits.

Furthermore, the invention provides a kit for concentrating, isolating and/or purifying nucleic acids in a method according to the present invention comprising: a) a chaotrope-free binding mediating agent, preferably a binding mediating agent as described above, and b) a solid phase capable of selectively binding the nucleic acids in the presence of the binding mediating agent, preferably a solid phase as described above.

The kit, furthermore, may comprise one or more washing liquid(s)/washing buffer(s), preferably selected from the group as described above; and/or at least one elution buffer/solvent preferably any elution buffer/solvent as described above, and/or instructions for using the kit.

### Example

50 mL of human urine were mixed with 2 mL of a 0.5 % (w/v) CTAB solution in water and filtered through a folded filter. The filtered solution was applied to a spin column containing a silica membrane (Compact prep GIGA Qiagen, Hilden, Germany) by means of reduced pressure. The column was washed in a first step using 20 mL of water and in a second step with 50 mL of buffer PE (Qiagen, Hilden, Germany). The column was dried for 3 min by centrifuging of the column for 5 minutes at 5.000 xg. The nucleic acids were eluted by applying 1 mL of buffer TE (Qiagen, Hilden, Germany) and spinning the column at 5.000 xg for 5 min. The eluate collected in this elution step was re-applied to the column and forced through the column by spinning it once again.

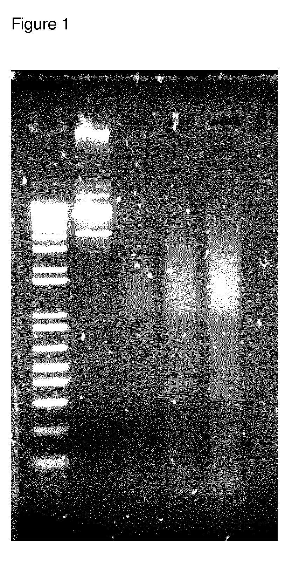

The eluate collected from the second centrifuging step was than mixed with 2 mL of a 0.5 % (w/v) CTAB solution in water and applied to a Compact prep microspin column. (Qiagen, Hilden, Germany) The liquid phase was removed by vacuum. The nucleic acids were eluted by applying 100 µL buffer TE to the column, spinning the column at 5,000 xg for 5 min collecting the eluate, reapplying it to the column and spinning as described above. The eluates were than analysed on an agarose gel (0.8 %, 50 mL, using 2,5 µL GelStar® by Combrex BioScience Rockland Inc., Rockland, ME, USA as a stain) run at 100 V for 50 min. The gel is presented in figure 1. In the lane on the very left, a 1 kb size ladder (Life Technologies) is shown as a reference. In the second lane a control sample of pQ51 is shown. 2 µg of said plasmid pQ51 were diluted in 1 mL of water and the sample was applied to a Compact prep microspin column by means of reduced pressure. The column was washed using 800 µL of buffer PE, the washing buffer being removed by spinning the column at 5,000 xg for 3 min. The nucleic acids were eluted by applying 100 µL of buffer TE and spinning the column for three minutes at 10,000 xg. The eluate was collected and 2 µL of this eluate were applied in lane 2 of the gel shown in figure 1. In the remaining three lanes, 5, 10 and 15 µL (from left to right) of the sample eluate obtained as described above were analysed. It can be seen, that the method of the present invention is suitable for concentrating, isolating and analyzing even short fragments of free-circulating DNA from large sample volumes, in which these nucleic acids are present in a very low concentration.

## Claims

1. Method for concentrating, isolating and/or purifying nucleic acids from an aqueous sample comprising said nucleic acids in a concentration of less than 500 ng/mL, preferably less than 250 ng/mL, more preferably less than 150 ng/mL and most preferably equal to or less than 100 ng/mL, said method comprising a step of separating the nucleic acids from the aqueous sample by contacting the sample with a binding mediating agent and a solid phase which selectively binds the nucleic acids in the presence of the binding mediating agent,
wherein neither the binding mediating agent nor any other compound added to the sample comprises a chaotropic substance, selected from the group comprising sodium perchloride, sodium iodide, potassium iodide, guanidinium thionate, guamidinium isothiocyanate and guanidine hydrochloride.

2. The method according to claim 1, comprising the steps of
a) optionally separating any solids from the sample, preferably by centrifugation or filtration,
b) contacting the sample with the solid phase, which binds the nucleic acids in the presence of the binding mediating agent,
c) optionally incubating the sample in contact with the solid phase,
d) separating the solid phase essentially from any remaining parts of the sample not bound to the solid phase,
e) optionally washing the solid phase,
f) optionally drying the solid phase, and
g) eluting the nucleic acid from the solid phase.

3. The method according to claim 1 or 2, further comprising the step of mixing the sample with said binding mediating agent before or while contacting the sample with the solid phase.

4. The method according to any of claims 1 to 3, therein the binding mediating agent comprises at least one organic cation, preferably selected from the group comprising cationic polyamines and cationic surfactants, more preferably selected from the spermine and spermidine, and cationic surfactants of the general structure R¹R²R³R⁴N⁺X⁻, wherein R¹, R², R³, and R⁴ independently represent a C₁-C₂₀ alkyl group and X⁻ represents a negatively charged counter ion.

5. The method according to claim 4, wherein the organic cation is a cationic surfactant of the formula R¹R²R³R⁴N⁺X⁻, wherein R¹ represents a linear C₈-C₁₆-alkyl chain and R², R³, and R⁴ independently represent Me or Et, preferably selected from the group comprising octyltrimethylammonium bromide (OTAB), dodecyltrimethylammonium bromide (DTAB), tetradecyltrimethylammonium bromide (TTAB), and cetyltrimethylammonium bromide (CTAB), or a mixture thereof.

6. The method according to any of claims 1 to 5, wherein the concentration of the binding mediating agent in the sample is in the range of from 0.001 to 5 wt-%, preferably of from 0.05 to 1 wt-%, and most preferably of from 0.01 to 0.2 wt-%.

7. The method according to any of claims 1 to 6, wherein the solid phase is selected from the group comprising beads, including magnetic beads, particulates, powders, layers, meshes, tips, dipsticks, tubes, container walls, filters, gels, ion exchange resins, reversed phase resins, matrices, and membranes, preferably selected from the group comprising polystyrene or silica beads, magnetic beads coated with a polystyrene or silica layer, polystyrene or silica beads further functionalized with negatively charged polymers, polystyrene or silica slides, polystyrene or silica powder, quartz filter paper, quartz wool, polystyrene or silica membranes, and polystyrene or silica matrices, and most preferably represents a silica membrane or a silica matrix.

8. The method according to any of claims 2 to 7, wherein at least one, preferably at least two steps of washing according to step e) are carried out, preferably by using washing liquid(s)/washing buffer(s) selected from the group comprising water, aqueous solutions comprising 0.01 to 25 % of a C₁-C₅-alcohol, a buffering agent, a chelating agent, or mixtures thereof.

9. A method according to any of claims 2 to 8, wherein the binding mediating agent represents CTAB and the step of eluting the nucleic acids from the solid phase according to step g) is carried out using an aqueous salt solution having a conductivity of at least 60 mS/m or alcoholic solutions comprising at least 30 % (v/v) of an alcohol, preferably ethanol.

10. Use of a cationic surfactant of the formula R¹R²R³R⁴N⁺X⁻, wherein R¹ represents a linear C₈-C₁₆-alkyl chain and R², R³, and R⁴ independently represent Me or Et, preferably selected from the group comprising octyltrimethylammonium bromide (OTAB), dodecyltrimethylammonium bromide (DTAB), tetradecyltrimethylammonium bromide (TTAB), and cetyltrimethylammonium bromide (CTAB) or a cationic polyamine, preferably selected from spermine and spermidine, or a mixture in a method for concentrating, isolating, and/or purifying nucleic acids from an aqueous liquid, comprising said nucleic acids in a concentration of less than 500 ng/mL, preferably less than 250 ng/mL, more preferably less than 150 ng/mL and most preferably equal to or less 100 ng/mL.

11. Method according to any of claims 1 to 9 or use according to claim 10, wherein the nucleic acids are circulating nucleic acids, preferably free-circulating nucleic acids and most preferably free-circulating DNA.

12. Method according to any of claims 1 to 9 or 11 or use according to claim 10 or 11, wherein the aqueous liquid is a cell-free body fluid, preferably plasma, serum, liquor, urine or tears liquid.

13. Method according to any of claims 1 to 9, 11 or 12 or use according to any of claims 10 to 12, wherein the length of the nucleic acids to be concentrated, isolated and/or purified is in the range of from 5 bp (DNA)/nt (RNA) to genomic DNA, preferably from 10 bp (DNA)/nt (RNA) to 10.000 bp (DNA)/nt(RNA), preferably of from 20 bp (DNA)/nt (RNA) to 5.000 bp (DNA)/nt(RNA).

14. A kit for concentrating, isolating and/or purifying nucleic acids in a method according to any of claims 1 to 9 or 11 to 13, comprising:
a) a chaotrope-free binding mediating agent, preferably a binding mediating agent according to claims 4 or 5, and
b) a solid phase capable of selectively binding the nucleic acids in the presence of the binding mediating agent, preferably a solid phase according to claim 7.

15. A kit according to claim 14, further comprising at least one of:
c) one or more washing liquid(s)/washing buffer(s), preferably selected from the group mentioned in claim 8,
d) at least one elution buffer/solvent, preferably an elution buffer/solvent according to claim 9, and/or
e) instructions for using the kit.
